(19) Europäisches Patentamt
European Patent Office
Office européen des brevets

(11) **EP 3 877 732 B1**

(12) **EUROPÄISCHE PATENTSCHRIFT**

(45) Veröffentlichungstag und Bekanntmachung des Hinweises auf die Patenterteilung:
**16.08.2023   Patentblatt 2023/33**

(21) Anmeldenummer: **19729510.8**

(22) Anmeldetag: **05.06.2019**

(51) Internationale Patentklassifikation (IPC):
**G01F 23/296** *(2022.01)*    **G01N 9/24** *(2006.01)*
**G01N 11/00** *(2006.01)*

(52) Gemeinsame Patentklassifikation (CPC):
**G01F 23/2966; G01N 9/24;** G01N 2011/0073

(86) Internationale Anmeldenummer:
**PCT/EP2019/064724**

(87) Internationale Veröffentlichungsnummer:
**WO 2020/094266 (14.05.2020 Gazette 2020/20)**

(54) **VIBRONISCHER MULTISENSOR**

VIBRONIC MULTISENSOR

MULTICAPTEUR VIBRONIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.11.2018   DE 102018127526**

(43) Veröffentlichungstag der Anmeldung:
**15.09.2021   Patentblatt 2021/37**

(73) Patentinhaber: **Endress+Hauser SE+Co. KG**
**79689 Maulburg (DE)**

(72) Erfinder:
• **LOPATIN, Sergey**
**79540 Lörrach (DE)**

• **SCHLEIFERBRÖCK, Jan**
**79595 Rümmingen (DE)**
• **BRENGARTNER, Tobias**
**79312 Emmendingen (DE)**

(74) Vertreter: **Koslowski, Christine Adelheid**
**Endress+Hauser Group Services**
**(Deutschland) AG+Co. KG**
**Colmarer Straße 6**
**79576 Weil am Rhein (DE)**

(56) Entgegenhaltungen:

| | |
|---|---|
| WO-A1-2013/152302 | DE-A1-102016 109 250 |
| DE-A1-102016 114 974 | US-A- 5 369 600 |
| US-A1- 2010 083 750 | US-A1- 2015 338 262 |
| US-A1- 2018 024 097 | US-A1- 2018 224 318 |

EP 3 877 732 B1

**Beschreibung**

Die Erfindung betrifft ein Verfahren zur Bestimmung und/oder Überwachung von zumindest zwei verschiedenen Prozessgrößen eines Mediums mittels einer Vorrichtung umfassend eine Sensoreinheit mit zumindest einer mechanisch schwingfähigen Einheit und einem ersten und einem zweiten piezoelektrischen Element. Die Erfindung betrifft ferner eine Vorrichtung, welche zur Durchführung eines erfindungsgemäßen Verfahrens ausgestaltet ist. Das Medium befindet sich in einem Behältnis, beispielsweise in einem Behälter oder in einer Rohrleitung.

Vibronische Sensoren finden vielfach Anwendung in der Prozess- und/oder Automatisierungstechnik. Im Falle von Füllstandsmessgeräten weisen sie zumindest eine mechanisch schwingfähige Einheit, wie beispielsweise eine Schwinggabel, einen Einstab oder eine Membran auf. Diese wird im Betrieb mittels einer Antriebs-/Empfangseinheit, häufig in Form einer elektromechanischen Wandlereinheit, zu mechanischen Schwingungen angeregt, welche wiederum beispielsweise ein piezoelektrischer Antrieb oder ein elektromagnetischer Antrieb sein kann. Entsprechende Feldgeräte werden von der Anmelderin in großer Vielfalt hergestellt und beispielsweise unter der Bezeichnung LIQUIPHANT oder SOLIPHANT vertrieben. Die zugrundeliegenden Messprinzipien sind im Prinzip aus einer Vielzahl von Veröffentlichungen bekannt. Die Antriebs-/Empfangseinheit regt die mechanisch schwingfähige Einheit mittels eines elektrischen Anregesignals zu mechanischen Schwingungen an. Umgekehrt kann die Antriebs-/Empfangseinheit die mechanischen Schwingungen der mechanisch schwingfähigen Einheit empfangen und in ein elektrisches Empfangssignal umwandeln. Bei der Antriebs-/Empfangseinheit handelt es sich entsprechend entweder um eine separate Antriebseinheit und eine separate Empfangseinheit, oder um eine kombinierte Antriebs-/Empfangseinheit.

Dabei ist die Antriebs-/Empfangseinheit in vielen Fällen Teil eines rückgekoppelten elektrischen Schwingkreises, mittels welchem die Anregung der mechanisch schwingfähigen Einheit zu mechanischen Schwingungen erfolgt. Beispielsweise muss für eine resonante Schwingung die Schwingkreisbedingung, gemäß welcher der Verstärkungsfaktor $\geq 1$ ist und alle im Schwingkreis auftretenden Phasen ein Vielfaches von 360° ergeben, erfüllt sein. Zur Anregung und Erfüllung der Schwingkreisbedingung muss eine bestimmte Phasenverschiebung zwischen dem Anregesignal und dem Empfangssignal gewährleistet sein. Deshalb wird häufig ein vorgebbarer Wert für die Phasenverschiebung, also ein Sollwert für die Phasenverschiebung zwischen dem Anregesignal und dem Empfangssignal eingestellt. Hierfür sind aus dem Stand der Technik unterschiedlichste Lösungen, sowohl analoge als auch digitale Verfahren, bekannt geworden, wie beispielsweise in den Dokumenten DE102006034105A1, DE102007013557A1, DE102005015547A1, DE102009026685A1, DE102009028022A1, DE102010030982A1 oderDE00102010030982A1 beschrieben. US2018/224318A1 beschreibt einen Sensor mit einer mechanisch schwingfähigen Einheit und einer elektromagnetisch schwingfähigen Einheit mit einem aktiven Element zur Erzeugung elektromagnetischer Schwingungen. Bei diesem Dokument wird nicht das Ultraschallprinzip angewendet.

Sowohl das Anregesignal als auch das Empfangssignal sind charakterisiert durch ihre Frequenz $\omega$, Amplitude A und/oder Phase $\Phi$. Entsprechend werden Änderungen in diesen Größen üblicherweise zur Bestimmung der jeweiligen Prozessgröße herangezogen. Bei der Prozessgröße kann es sich beispielsweise um einen Füllstand, einen vorgegebenen Füllstand, oder auch um die Dichte oder die Viskosität des Mediums, sowie um den Durchfluss handeln. Bei einem vibronischen Grenzstandschalters für Flüssigkeiten wird beispielsweise unterschieden, ob die schwingfähige Einheit von der Flüssigkeit bedeckt ist oder frei schwingt. Diese beiden Zustände, der Freizustand und der Bedecktzustand, werden dabei beispielsweise anhand unterschiedlicher Resonanzfrequenzen, also anhand einer Frequenzverschiebung, unterschieden.

Die Dichte und/oder Viskosität wiederum lassen sich mit einem derartigen Messgerät nur ermitteln, wenn die schwingfähige Einheit vom Medium bedeckt ist. Im Zusammenhang mit der Bestimmung der Dichte und/oder Viskosität sind ebenfalls unterschiedliche Möglichkeiten aus dem Stand der Technik bekannt geworden, wie beispielswiese die in den Dokumenten DE10050299A1, DE102007043811A1, DE10057974A1, DE102006033819A1, DE102015102834A1 oder DE102016112743A1 offenbaren.

Mit einem vibronischen Sensor lassen sich entsprechend mehrere Prozessgrößen bestimmen und für eine Charakterisierung des jeweiligen Prozesses heranziehen. In vielen Fällen werden für eine umfassende Prozessüberwachung und/oder -kontrolle allerdings weitere Informationen über den Prozess, insbesondere Kenntnis über weitere physikalische und/oder chemische Prozessgrößen und/oder -parameter benötigt. Dies kann beispielsweise durch die Integration weiterer Feldgeräte in den jeweiligen Prozess erreicht werden. Dann können die von den verschiedenen Messgeräten zur Verfügung gestellten Messwerte in einer den Geräten übergeordneten Einheit geeignet weiterverarbeitet werden.

Nun ist es aber so, dass die unterschiedlichen Messgeräte zum einen über unterschiedliche Messgenauigkeiten verfügen. Darüber hinaus können Drift- und/oder Alterungseffekte jeweils sehr unterschiedlich sein. Solche Effekte können aber die jeweilige Messung bzw. Prozessüberwachung und/oder -kontrolle erheblich erschweren bzw. ungenau machen. Darüber hinaus kann es schwierig sein, den jeweiligen Zustand der einzelnen Feldgeräte jeweils im fortlaufenden Betrieb festzustellen. Somit liegt der vorliegenden Erfindung die Aufgabe zugrunde, die Funktionalität eines vibronischen Sensors zu erweitern.

Diese Aufgabe wird gelöst durch das Verfahren nach Anspruch 1 sowie durch die Vorrichtung nach Anspruch 13.

Hinsichtlich des Verfahrens wird die Aufgabe gelöst durch ein Verfahren zur Bestimmung und/oder Überwachung von zumindest zwei verschiedenen Prozessgrößen eines Mediums, wobei

- eine Sensoreinheit mittels eines Anregesignals zu mechanischen Schwingungen angeregt wird,
- die mechanischen Schwingungen von der Sensoreinheit empfangen und in ein erstes Empfangssignal umgewandelt werden,
- von der Sensoreinheit ein Sendesignal ausgesendet und ein zweites Empfangssignal empfangen wird, und
- anhand des ersten Empfangssignals eine erste Prozessgröße und anhand des zweiten Empfangssignals eine zweite Prozessgröße ermittelt wird.

Die Sensoreinheit ist Teil einer Vorrichtung zur Bestimmung und/oder Überwachung von zumindest zwei verschiedenen Prozessgrößen eines Mediums und umfasst eine mechanisch schwingfähige Einheit sowie zumindest ein erstes und ein zweites piezoelektrisches Element. Bei der mechanisch schwingfähigen Einheit handelt es sich beispielsweise um eine Membran, einen Einstab, eine Anordnung von zumindest zwei Schwingelementen, oder um eine Schwinggabel. Die beiden piezoelektrischen Elemente können darüber hinaus zumindest teilweise als Antriebs-/Empfangseinheit zur Erzeugung der mechanischen Schwingungen der mechanisch schwingfähigen Einheit dienen.

Das Sendesignal kann ferner von einem der beiden piezoelektrischen Elemente ausgesendet und von dem jeweils anderen piezoelektrischen Element in Form des zweiten Empfangssignals empfangen werden. Das Sendesignal durchläuft zumindest zeitweise und abschnittsweise das Medium und wird durch die physikalischen und/oder chemischen Eigenschaften des Mediums beeinflusst und kann entsprechend zur Bestimmung einer zweiten Prozessgröße des Mediums herangezogen werden.

Im Rahmen der vorliegenden Erfindung ist es vorteilhaft möglich, zumindest zwei Messprinzipien in einer einzigen Vorrichtung zu realisieren. Die Sensoreinheit führt einerseits mechanische Schwingungen aus; zudem wird ein Sendesignal ausgesendet. In Reaktion auf die mechanischen Schwingungen und auf das Sendesignal werden zwei Empfangssignale empfangen und hinsichtlich zumindest zwei unterschiedlicher Prozessgrößen ausgewertet. Die beiden Empfangssignale können dabei vorteilhaft unabhängig voneinander ausgewertet werden. So kann erfindungsgemäß die Anzahl ermittelbarer Prozessgrößen deutlich erhöht werden, was zu einer höheren Funktionalität des jeweiligen Sensors bzw. in einem erweiterten Anwendungsbereich resultiert.

In einer Ausgestaltung der Erfindung wird die Sensoreinheit gleichzeitig mittels des Anregesignals und mittels des Sendesignals beaufschlagt wird, wobei das Anregesignal und das Sendesignal einander überlagert werden. Alternativ kann die Sensoreinheit aber auch abwechselnd mittels des Anregesignal und mittels des Sendesignals beaufschlagt werden.

Eine bevorzugte Ausgestaltung beinhaltet, dass es sich bei dem Anregesignal um ein elektrisches Signal mit zumindest einer vorgebbaren Frequenz, insbesondere um ein sinusförmiges oder um ein rechteckförmiges Signal. Vorzugsweise wird die mechanisch schwingfähige Einheit zumindest zeitweise zu Resonanzschwingungen angeregt. Die mechanischen Schwingungen werden durch das die schwingfähige Einheit umgebende Medium beeinflusst, so dass anhand eines die Schwingungen repräsentierenden Empfangssignals Rückschlüsse auf verschiedene Eigenschaften des Mediums möglich sind.

Eine weitere besonders bevorzugte Ausgestaltung beinhaltet, dass es sich bei dem Sendesignal um ein, insbesondere gepulstes, Ultraschallsignal, insbesondere um zumindest einen Ultraschallpuls, handelt. Als zweites angewendetes Messverfahren wird demnach im Rahmen der vorliegenden Erfindung eine Ultraschall-basierte Messung durchgeführt. Das jeweils ausgesendete Sendesignal durchläuft zumindest teilweise das Medium und wird von diesem in seinen Eigenschaften beeinflusst. Entsprechend können anhand des jeweils empfangenen zweiten Empfangssignals ebenfalls Rückschlüsse auf verschiedene Medien gezogen werden.

Vorteilhaft lassen sich mit den beiden angewendeten Verfahren zumindest teilweise unterschiedliche Prozessgrößen und/oder -parameter mit einer Vorrichtung unabhängig voneinander bestimmen, so dass eine umfassende Analyse des jeweiligen Prozesses mittels eines einzigen Messgeräts ermöglicht wird. Dadurch, dass dieselbe Sensoreinheit für beide Messverfahren zum Einsatz kommt, kann darüber hinaus die Genauigkeit der Messungen deutlich erhöht werden. Darüber hinaus kann anhand der beiden Prozessgrößen eine Zustandsüberwachung der Vorrichtung vorgenommen werden. In dieser Hinsicht sind zahlreiche Ausgestaltungen möglich, von denen einige bevorzugte Varianten nachfolgend angegeben werden.

Eine besonders bevorzugte Ausgestaltung beinhaltet, dass es sich bei der ersten Prozessgröße um die Dichte des Mediums und bei der zweiten Prozessgröße um die Schallgeschwindigkeit innerhalb des Mediums oder eine daraus abgeleitete Größe handelt. Es wird also anhand des ersten Empfangssignals die Dichte des Mediums und anhand des zweiten Empfangssignals die Schallgeschwindigkeit innerhalb des Mediums bestimmt.

Hierbei ist es von Vorteil, wenn anhand der Schallgeschwindigkeit ein Referenzwert für die Dichte ermittelt wird, und wobei der Referenzwert mittels eines aus dem ersten Empfangssignals ermittelten Wertes für die Dichte verglichen wird. Vorzugsweise wird anhand der aus dem zweiten Empfangssignal ermittelten Schallgeschwindigkeit eine Konzentration einer in einem Referenzmedium gelösten Referenzsubstanz in einem vorgebbaren Behälter ermittelt. Aus der Konzentration kann anschließend der Referenzwert für die Dichte des Referenzmediums ermittelt werden. Zudem kann ein Messwert für die Dichte aus dem ersten Empfangssignal ermittelt werden. Die beiden Werte für die Dichte können dann miteinander verglichen werden. Insbesondere kann der aus dem ersten Empfangssignal ermittelte Wert für die Dichte anhand des aus dem zweiten Empfangssignals ermittelten Referenzwerts für die Dichte justiert werden. Auf diese Weise kann eine nachteilige Beeinflussung der Geometrie des jeweils verwendeten Behältnisses auf die vibronische Bestimmung der Dichte kompensiert werden.

Darüber hinaus ist es von Vorteil, wenn zumindest eine dritte Prozessgröße, insbesondere die Viskosität, des Mediums bestimmt wird. Es versteht sich jedoch von selbst, dass neben den hier explizit genannten Prozessgrößen auch weitere Prozessgrößen und/oder -parameter, welche mittels der beiden durchgeführten Messungen zugänglich sind, ebenfalls bestimmt und für eine Charakterisierung des jeweiligen Prozesses herangezogen werden können.

In einer Ausgestaltung des Verfahrens wird anhand des ersten und zweiten Empfangssignals und/oder anhand der ersten und zweiten Prozessgröße ermittelt, ob sich ein Ansatz an der Sensoreinheit gebildet hat. Die beiden Empfangssignale verhalten sich üblicherweise jeweils unterschiedlich in Abhängigkeit eines Ansatzes im Bereich der Sensoreinheit. Das Vorhandensein eines Ansatzes kann entsprechend beispielsweise anhand einer zeitlichen Betrachtung der beiden Empfangssignale und/oder Prozessgrößen festgestellt werden.

In einer weiteren Ausgestaltung des Verfahrens wird anhand des ersten und zweiten Empfangssignals und/oder anhand der ersten und zweiten Prozessgröße eine Drift und/oder eine Alterung der Sensoreinheit ermittelt. Auch in diesem Zusammenhang kann beispielsweise eine zeitliche Betrachtung des ersten und zweiten Empfangssignals und/oder der ersten und zweiten Prozessgröße vorgenommen werden.

Eine besonders bevorzugte Ausgestaltung sieht vor, dass das erste und zweite Empfangssignal, die erste und zweite Prozessgröße und/oder ein zeitlicher Verlauf des ersten und zweiten Empfangssignals und/oder der ersten und zweiten Prozessgröße miteinander verglichen werden. Aus dem Vergleich kann dann auf das Vorhandensein eines Ansatzes, auf eine Drift oder eine Alterung der Sensoreinheit geschlossen werden. Da zumindest zwei Empfangssignale bzw. Prozessgrößen zugänglich sind, kann eine hohe Genauigkeit hinsichtlich der jeweils getroffenen Aussagen über einen Ansatz, eine Drift oder eine Alterung erzielt werden.

Durch die erfindungsgemäße Realisierung zweier unterschiedlicher Messungen mit einer einzigen Sensoreinheit kann entsprechend das Vorhandensein von Ansatz, oder auch eine Drift oder eine Alterung der Sensoreinheit zuverlässig erkannt werden.

In einer weiteren, besonders bevorzugten Ausgestaltung wird bei der Bestimmung und/oder Überwachung zumindest einer Prozessgröße oder bei der Bestimmung einer aus zumindest einer Prozessgröße und/oder zumindest einem Empfangssignal abgeleiteten Größe ein Einfluss eines Ansatzes einer Drift und/oder einer Alterung der Sensoreinheit auf das erste und/oder zweite Empfangssignal reduziert oder kompensiert. Der Einfluss eines Ansatzes, einer Drift und/oder Alterung der Sensoreinheit kann demnach bei der Bestimmung und/oder Überwachung der jeweiligen Prozessgröße berücksichtigt werden, so dass die jeweilige Prozessgröße ohne auf dem Vorhandensein eines Ansatzes, einer Drift und/oder Alterung bestimmt werden kann. Zur Reduzierung oder zur Kompensation des Einflusses kann beispielsweise ein geeigneter Algorithmus hinterlegt werden, anhand dessen ein nicht durch den Einfluss des Ansatzes, der Drift und/oder Alterung der Sensoreinheit verfälschter Wert für die jeweilige Prozessgröße ermittelbar ist. Somit kann eine verbesserte Messgenauigkeit erreicht werden.

Noch eine besonders bevorzugte Ausgestaltung beinhaltet, dass anhand des ersten und zweiten Empfangssignals und/oder anhand der ersten und zweiten Prozessgröße eine erste Konzentration einer ersten in dem Medium enthaltenen Substanz und eine zweite Konzentration einer zweiten in dem Medium enthaltenen Substanz ermittelt wird. Gemäß Stand der Technik sind für eine derartige Analyse des Mediums auf zwei unterschiedliche Substanzen hin in der Regel zwei separate Messgeräte erforderlich, welche unterschiedliche Messgrößen bereitstellen. Erfindungsgemäß kann dagegen mittels einer einzigen Vorrichtung zuverlässig eine Aussage über zwei unterschiedliche Komponenten in einem Medium getätigt werden.

Eine bevorzugte Verwendung des Verfahrens betrifft die Überwachung eines Gärprozesses. Bei einer Gärung wird Zucker in Ethanol umgewandelt. Um eine qualitative Überwachung gewährleisten zu können, ist es deshalb erforderlich, sowohl die Konzentration von Zucker als auch von Ethanol zu bestimmen. Dies ist im Rahmen der vorliegenden Erfindung möglich.

Die der Erfindung zugrunde liegende Aufgabe wird ferner gelöst durch eine Vorrichtung zur Bestimmung und/oder Überwachung einer ersten und einer zweiten Prozessgröße eines Mediums, welche Vorrichtung dazu ausgestaltet ist, ein Verfahren nach zumindest einer der beschriebenen Ausgestaltungen auszuführen.

Es ist von Vorteil, wenn die Sensoreinheit eine mechanisch schwingfähige Einheit, und zumindest ein erstes, insbesondere zumindest ein erstes und ein zweites piezoelektrisches Element, umfasst. Es können aber auch mehr als zwei piezoelektrische Elemente vorhanden sein, die an unterschiedlichen Positionen relativ zur schwingfähigen Einheit angeordnet sein können.

So beinhaltet eine bevorzugte Ausgestaltung, dass die mechanisch schwingfähige Einheit eine Schwinggabel mit einem ersten und einem zweiten Schwingelement ist, wobei das erste piezoelektrische Element zumindest teilweise in einem der beiden Schwingelemente angeordnet ist, insbesondere wobei das erste piezoelektrische Element zumindest teilweise in dem ersten Schwingelement und das zweite piezoelektrische Element zumindest teilweise in dem zweiten Schwingelement angeordnet ist. Entsprechende Ausgestaltungen einer Sensoreinheit sind beispielsweise in den Dokumenten DE102012100728A1 sowie in der bisher unveröffentlichten deutschen Patentanmeldung mit dem Aktenzeichen DE102017130527A1 beschrieben worden. Auf beide Anmeldungen wird im Rahmen der vorliegenden Erfindung vollumfänglich Bezug genommen. Es sei darauf verwiesen, dass die vorliegende Erfindung jedoch nicht auf eine der in den beiden Dokumenten beschriebenen möglichen Ausgestaltungen der Sensoreinheit beschränkt ist. Hierbei handelt es sich nur um beispielhafte mögliche konstruktive Ausgestaltungen der Sensoreinheit, die sich zur Durchführung des erfindungsgemäßen Verfahrens eignen. So ist beispielsweise auch die Verwendung eines einzigen piezoelektrischen Elements, welches beispielsweise in einem der beiden Schwingelemente angeordnet sein kann, ausreichend. Auch ist es nicht zwingend notwendig, die piezoelektrischen Elemente ausschließlich im Bereich der Schwingelemente anzuordnen. Vielmehr können einzelne der verwendeten piezoelektrischen Elemente auch im Bereich der Membran oder in weiteren nicht für die vibronische Anregung verwendeten Schwingelementen, welche ebenfalls auf der Membran aufgebracht sind, angeordnet sein.

Es sei ferner darauf verwiesen, dass die im Zusammenhang mit dem erfindungsgemäßen Verfahren beschriebenen Ausgestaltungen sich mutatis mutandis auch auf die erfindungsgemäße Vorrichtung anwenden lassen und umgekehrt.

Die Erfindung wird anhand der nachfolgenden Figuren näher erläutert. Es zeigt:

Fig. 1: eine schematische Skizze eines vibronischen Sensors gemäß Stand der Technik,

Fig. 2 mehrere mögliche an sich aus dem Stand der Technik bekannte Ausgestaltungen einer Sensoreinheit, welche zur Durchführung des erfindungsgemäßen Verfahrens geeignet sind, und

Fig. 3 eine Illustrierung einer Ausgestaltung des erfindungsgemäßen Verfahrens zur Detektion von Ansatz im Bereich der Sensoreinheit.

In den Figuren sind gleiche Elemente jeweils mit demselben Bezugzeichen versehen.

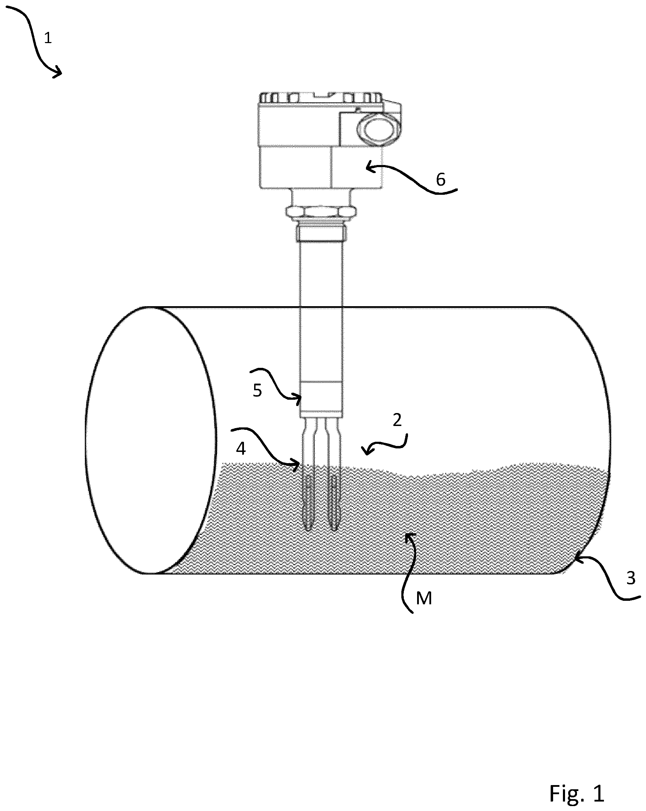

In Fig. 1 ist ein vibronischer Sensor 1 mit einer Sensoreinheit 2 gezeigt. Der Sensor verfügt über eine mechanisch schwingfähige Einheit 4 in Form einer Schwinggabel, welche teilweise in ein Medium M eintaucht, welches sich in einem Behälter 3 befindet. Die schwingfähige Einheit 4 wird mittels der Anrege-/Empfangseinheit 5 zu mechanischen Schwingungen angeregt, und kann beispielsweise durch einen piezoelektrischen Stapel- oder Bimorphantrieb sein. Andere vibronische Sensoren verfügen beispielsweise über elektromagnetische Antriebs-/Empfangseinheiten 5. Es ist sowohl möglich, eine einzige Antriebs-/Empfangseinheit 5 zu verwenden, welche zur Anregung der mechanischen Schwingungen sowie zu deren Detektion dient. Ebenso ist es aber denkbar, je eine Antriebseinheit und eine Empfangseinheit zu realisieren. Dargestellt ist in Fig. 1 ferner eine Elektronikeinheit 6, mittels welcher die Signalerfassung, -auswertung und/oder-speisung erfolgt.

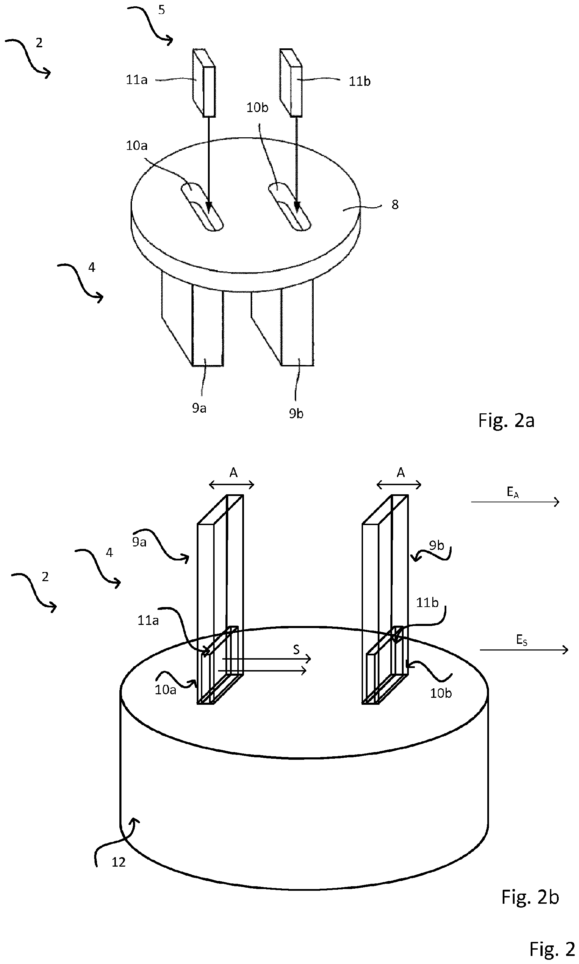

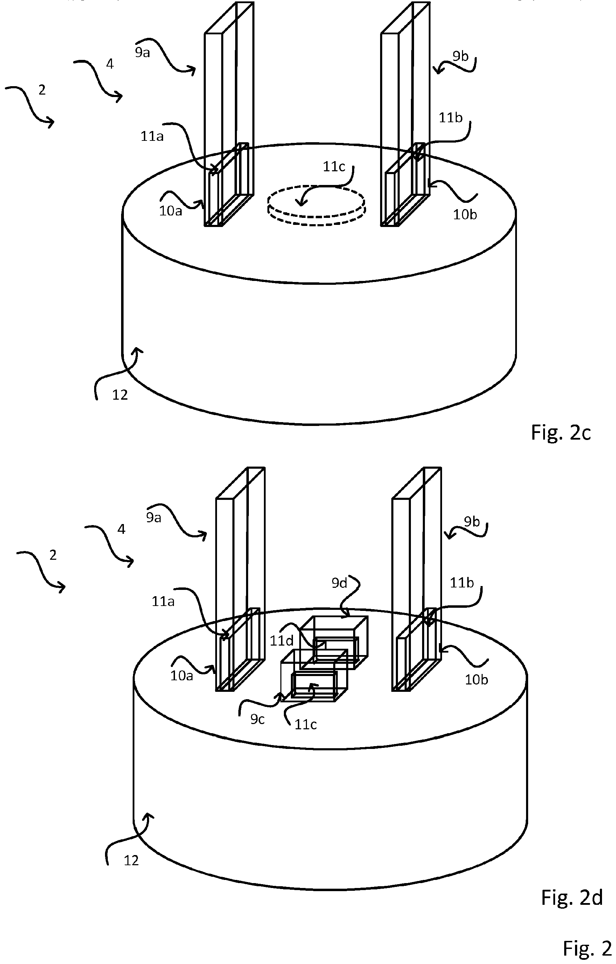

In Fig. 2 sind beispielhaft verschiedene Sensoreinheiten 2 gezeigt, welche sich zur Durchführung eines erfindungsgemäßen Verfahrens eignen. Die in Fig. 2a gezeigte mechanisch schwingfähige Einheit 4 umfasst zwei an einer Basis 8 angebrachte Schwingelemente 9a,9b, welche mithin auch als Gabelzinken bezeichnet werden. Optional kann an den Endseiten der beiden Schwingelemente 9a,9b außerdem jeweils ein Paddel angeformt sein [hier nicht gezeigt]. In jedem der beiden Schwingelemente 9a,9b ist jeweils ein, insbesondere taschenartiger, Hohlraum 10a, 10b eingebracht, in welchem jeweils zumindest ein piezoelektrisches Element 11a, 11b der Antriebs-/Empfangseinheit 5 angeordnet ist. Vorzugsweise sind die piezoelektrischen Elemente 11a und 11b innerhalb der Hohlräume 10a und 10b vergossen. Die Hohlräume 10a, 10b können dabei so beschaffen sein, dass sich die beiden piezoelektrischen Elemente 11a, 11b vollständig oder teilweise im Bereich der beiden Schwingelemente 9a, 9b befinden Eine solche sowie ähnliche Anordnungen sind in der DE102012100728A1 ausführlich beschrieben.

Eine weitere beispielhafte, mögliche Ausgestaltung einer Sensoreinheit 2 ist in Fig. 2b dargestellt. Die mechanisch schwingfähige Einheit 4 verfügt über zwei parallel zueinander ausgerichtete, hier stabförmig ausgestaltete, auf einem scheibenförmigen Element 12 angebrachte, Schwingelemente 9a, 9b, welche getrennt voneinander zu mechanischen Schwingungen anregbar sind, und bei denen die Schwingungen ebenfalls getrennt voneinander empfangen und ausgewertet werden können.

Beide Schwingelemente 9a und 9b weisen jeweils einen Hohlraum 10a und 10b auf, in welchen im dem scheibenförmigen Element 12 zugewandten Bereich jeweils zumindest ein piezoelektrisches Element 11a und 11b angeordnet ist. Bezüglich der Ausgestaltung gemäß Fig. 2b sei wiederum ferner auf in die bisher unveröffentlichte deutsche Patentanmeldung mit dem Aktenzeichen DE102017130527A1 verwiesen.

Wie in Fig. 2b schematisch eingezeichnet, wird erfindungsgemäß die Sensoreinheit 2 einerseits mit einem Anregesignal A beaufschlagt, derart, dass die schwingfähige Einheit 4 zu mechanischen Schwingungen angeregt wird. Die Schwingungen werden dabei vermittels der beiden piezoelektrischen Elemente 11a und 11b erzeugt. Es ist sowohl denkbar, dass beide piezoelektrischen Elemente mit demselben Anregesignal A beaufschlagt werden, als auch eine Beaufschlagung des ersten Schwingelements 11a mittels eines ersten Anregesignals $A_1$ und des zweiten Schwingelements 11b mittels eines zweiten Anregesignals $A_2$. Ebenso ist es sowohl denkbar, dass anhand der mechanischen Schwingungen ein erstes Empfangssignal $E_A$, oder von jedem Schwingelement 9a,9b ein separates Empfangssignal $E_{A1}$ bzw. $E_{A2}$ empfangen wird.

Darüber hinaus wird vom ersten piezoelektrischen Element 11a ausgehend ein Sendesignal S ausgesendet, welches von dem zweiten piezoelektrischen Element 11b in Form eines zweiten Empfangssignals $E_S$ empfangen wird. Da die beiden piezoelektrischen Elemente 11a und 11b zumindest im Bereich der Schwingelemente 9a und 9b angeordnet sind, durchläuft das Sendesignal S das Medium M, sofern die Sensoreinheit 2 mit dem Medium M in Kontakt ist und wird entsprechend von den Eigenschaften des Mediums M beeinflusst. Vorzugsweise handelt es sich bei dem Sendesignal S um ein, insbesondere gepulstes, Ultraschallsignal, insbesondere um zumindest einen Ultraschallpuls. Ebenso ist es aber denkbar, dass das Sendesignal S von dem ersten piezoelektrischen Element 11a im Bereich des ersten Schwingelements 9a ausgesendet wird und an dem zweiten Schwingelement 9b reflektiert wird. In diesem Falle wird das zweite Empfangssignal Es vom ersten piezoelektrischen Element 11a empfangen. Das Sendesignal S durchläuft in diesem Falle das Medium M zweimal, was zu einer Verdoppelung einer Laufzeit $_T$ des Sendesignals S führt.

Neben diesen beiden gezeigten Ausgestaltungen einer erfindungsgemäßen Vorrichtung 1 sind noch zahlreiche weitere Varianten denkbar, welche ebenfalls unter die vorliegende Erfindung fallen. Beispielsweise ist es für die Ausgestaltungen gemäß der Figuren Fig. 2a und Fig. 2b möglich, lediglich ein piezoelektrisches Element 11a,11b zu verwenden und zumindest in einem der beiden Schwingelemente 9a, 9b anzuordnen. In diesem Falle dient das piezoelektrische Element 9a zur Erzeugung des Anregesignals, und des Sendesignals S, sowie zum Empfangen des ersten $E_1$ und zweiten Empfangssignals $E_2$. Das Sendesignal wird in diesem Falle an dem zweiten Schwingelement 9b ohne piezoelektrisches Element 11b reflektiert.

Eine weitere, beispielhafte Möglichkeit ist in Fig. 2c dargestellt. Hier ist ein drittes piezoelektrisches Element 11c im Bereich der Membran 12 vorgesehen. Das dritte piezoelektrische Element 11c dient der Erzeugung des Anregesignals A und zum Empfangen des ersten Empfangssignals $E_1$; das erste 11a und zweite piezoelektrische Element 11b dienen der Erzeugung des Sendesignals S bzw. dem Empfangen des zweiten Empfangssignals $E_2$. Alternativ ist es beispielsweise möglich, mit dem ersten 11a und/oder zweiten piezoelektrischen Element 11b das Anregesignal A und das Sendesignal S zu erzeugen sowie das zweite Empfangssignal $E_2$ zu empfangen, wobei das dritte piezoelektrische Element 11c zum Empfangen des ersten Empfangssignals $E_1$ dient. Ebenso ist es möglich, mit dem ersten 11a und/oder zweiten piezoelektrischen Element 11b das Sendesignal S und mit dem dritten piezoelektrischen Element 11c das Anregesignal A zu erzeugen und mit dem ersten 11a und/oder zweiten piezoelektrischen Element 11b das erste $E_1$ und/oder zweite Empfangssignal $E_2$ zu empfangen. Auch im Falle der Fig. 2c ist es für andere Ausgestaltungen möglich, auf das erste 11a oder zweite piezoelektrische Element 11b zu verzichten.

Noch eine mögliche Ausgestaltung der Vorrichtung 1 ist Gegenstand von Fig. 2d. Die Vorrichtung umfasst ausgehend von der Ausgestaltung aus Fig. 2b ein drittes 9c und ein viertes Schwingelement 9d. Diese dienen jedoch nicht einer Schwingungserzeugung. Vielmehr ist in den zusätzlichen Elemente 9c, 9d jeweils ein drittes 11c und viertes piezoelektrisches Element 11d angeordnet. In diesem Falle wird die vibronische Messung mittels der ersten beiden piezoelektrischen Elemente 11a, 11b und die Ultraschallmessung mittels der anderen beiden piezoelektrischen Elemente 11c,11d durchgeführt. Auch hier kann je Messprinzip auf ein piezoelektrisches Element, z. B. 11b und 11d verzichtet werden. Aus Symmetriegründen ist es dagegen vorteilhaft, stets zwei zusätzliche Schwingelemente 9c, 0d zu verwenden.

Erfindungsgemäß resultieren das erste $E_A$ und zweite Empfangssignal Es grundsätzlich aus unterschiedlichen Messverfahren und können unabhängig voneinander bezüglich unterschiedlicher Prozessgrößen $P_1$ und $P_2$ ausgewertet werden. Hierdurch ergibt sich eine höhere Genauigkeit hinsichtlich der Bestimmung der verschiedenen verfügbaren Prozessgrößen, sowie eine größere Anzahl an bestimmbaren Größen. Entsprechend wird eine umfassende und genaue Charakterisierung des jeweiligen Prozesses möglich.

Eine vorteilhafte Ausgestaltung des erfindungsgemäßen Verfahrens beinhaltet die Bestimmung der Konzentration zweier unterschiedlicher in dem Medium enthaltener Substanzen. Um eine erste Konzentration einer ersten Substanz und eine zweite Konzentration einer zweiten Substanz, welche beide in demselben Medium enthalten sind, bestimmen zu können, müssen unabhängig voneinander zwei verschiedene Prozessgrößen oder -parameter ermittelt werden. Erfindungsgemäß können die beiden notwendigen Prozessgrößen oder-parameter mittels zweier voneinander unabhängiger Messverfahren, jedoch mittels derselben Sensoreinheit bestimmt werden. Dies führt zu einer erhöhten Genauigkeit bezüglich der Bestimmung der beiden Konzentrationen und.

Eine in diesem Zusammenhang bevorzugte Anwendung besteht in der Überwachung eines Gärprozesses. Dabei wird Zucker in Ethanol umgesetzt. Eine beispielhafte Möglichkeit zur Bestimmung der beiden Konzentrationen und von Zucker und Ethanol besteht darin, die Dichte $\rho$ des Mediums M anhand des ersten Empfangssignals und die Schallgeschwindigkeit des Mediums anhand des zweiten Empfangssignals zu ermitteln.

Eine weitere bevorzugte Anwendung besteht in der Überwachung einer Zuckerinvertierung oder eines Invertzuckers. Dabei wird überwacht, zu welchem Anteil sich ein Zuckergemisch, in der Regel Haushaltszucker, in Glucose bzw. Fructose umgesetzt hat. Auch in diesem Falle können die beiden Konzentrationen von Glucose und Fructose anhand des ersten und zweiten Empfangssignals ermittelt werden.

Die Dichte $\rho$ kann beispielsweise anhand folgender Gleichung bestimmt werden:

$$\rho = \frac{1}{S} \cdot \left[ \left( \frac{F_0}{F_{Med}} \right)^2 - 1 \right]$$

Dabei ist $F_{Med}$ die Schwingungsfrequenz der schwingfähigen Einheit 4 im Medium M, $F_0$ die Referenzfrequenz der schwingfähigen Einheit 4 im Vakuum bzw. an Luft, und S beschreibt die Empfindlichkeit der Sensoreinheit 2. Die Schwingungsfrequenz der schwingfähigen Einheit 4 im Medium M $F_{Med}$ kann direkt anhand des ersten Empfangssignals $E_A$ ermittelt werden.

Die Schallgeschwindigkeit $v_M$ des Mediums M lässt sich wiederum aus dem Abstand L zwischen dem ersten 11a und zweiten piezoelektrischen Element 11b, welche als Sendeeinheit und Empfangseinheit dienen, sowie der Laufzeit $_\tau$ des Sendesignals S vom ersten 11a zum zweiten piezoelektrischen Element 11b nach folgender Gleichung ermitteln:

$$v_M = \frac{L}{\tau}$$

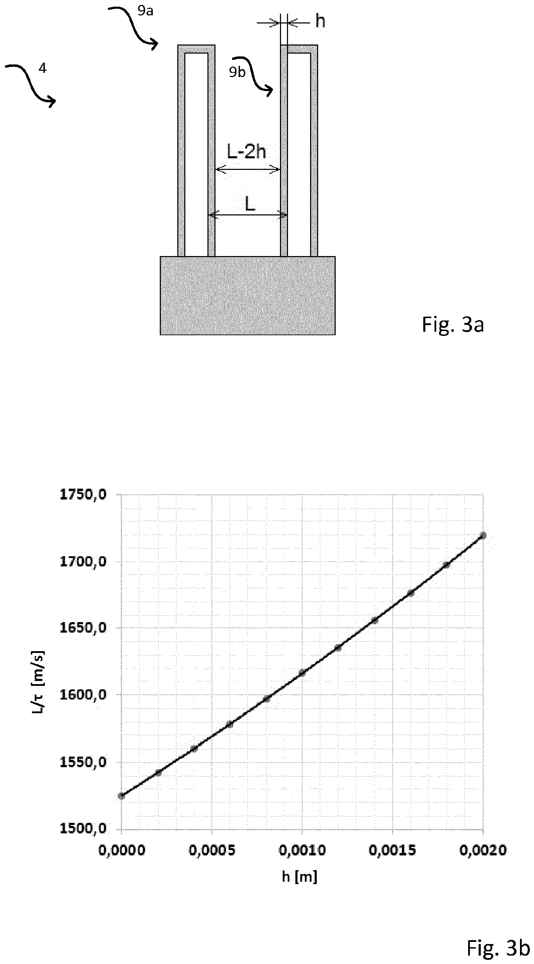

Die Abhängigkeit der Dichte $\rho$ und der Schallgeschwindigkeit $v_M$ sind in Fig. 3a illustriert. Fig. 3a zeigt zu diesem Zwecke eine schematische Zeichnung einer schwingfähigen Einheit 4 in Form einer Schwinggabel mit zwei im Abstand L voneinander angeordneten Schwingelementen 9a und 9b. Für die folgende Betrachtung sei ferner angenommen, dass sich im Bereich der Schwingelemente 9a und 9b ein Ansatz der Dicke h gebildet hat.

In Fig. 3b ist die anhand der gemessenen Laufzeit $_\tau$ und anhand des Abstands L zwischen den beiden Schwingelementen 9a und 9b berechnete Schallgeschwindigkeit $v_M$ für ein Medium M mit einer Dichte $\rho$ von 2,0 g/cm3 bei einer Temperatur von 20°C dargestellt. Mit zunehmenden Ansatz, bzw. mit zunehmender Dicke h des Ansatzes, steigt die gemessene Schallgeschwindigkeit vnn an.

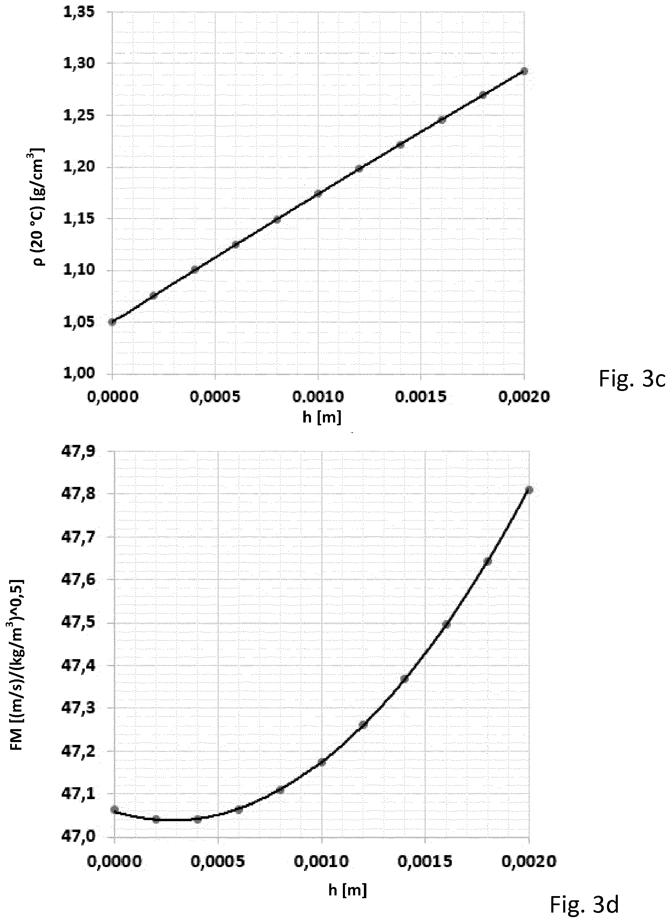

Fig. 3c zeigt wiederum die anhand der gemessenen Schwingfrequenz f der schwingfähigen Einheit 4 berechnete Dichte $\rho$ bei einer Temperatur von 20°C als Funktion der Ansatzdicke h. Auch die Dichte $\rho$ steigt mit zunehmender Dicke h des Ansatzes, allerdings sind die Steigungen der Dichte $\rho$ und der Schallgeschwindigkeit vnn in Abhängigkeit der Dicke h des Ansatzes jeweils unterschiedlich.

Im Folgenden wird ein bevorzugtes Ausführungsbeispiel zur Kompensation bzw. Reduzierung des Einflusses eines Ansatzes auf die Bestimmung einer Prozessgröße $P_1$-$P_3$ illustriert. Die nachfolgenden Überlegungen gelten dabei analog für den Fall, dass eine Drift und/oder Alterung der Sensoreinheit 2 auftritt. Es sei ferner darauf verwiesen, dass die hier beschriebene Kompensation des Einflusses von Ansatz lediglich eine von vielen Möglichkeiten darstellt, den Einfluss eines Ansatzes zu kompensieren. Die vorliegende Erfindung ist entsprechend keineswegs auf das nachfolgend angegebene Ausführungsbeispiel beschränkt.

Zur Kompensation des Einflusses eines Ansatzes kann eine aus zumindest einer Prozessgröße abgeleitete Größe FM ermittelt werden. Vorliegend wird anhand der Schallgeschwindigkeit $v_M$ und der Dichte $\rho$ die Größe FM gemäß

$$FM = \frac{v_m}{\rho^n}$$

bestimmt. Diese Größe ist in Fig. 3d für den Fall n=0,5 dargestellt. Wie aus der Grafik hervorgeht, ist der Einfluss eines Ansatzes der Dicke h auf die Größe FM vernachlässigbar. Berechnet man anhand der Größe FM eine Prozessgröße $P_1$-$P_3$, so erfolgt die Berechnung im Wesentlichen ohne den Einfluss eines Ansatzes.

**Bezugszeichenliste**

| | |
|---|---|
| 1 | Vibronischer Sensor |
| 2 | Sensoreinheit |
| 3 | Behälter |
| 4 | Schwingfähige Einheit |
| 5 | Antriebs-/Empfangseinheit |
| 6 | Elektronikeinheit |
| 8 | Basis |
| 9a, 9b | Schwingelemente |
| 10a, 10b | Hohlräume |
| 11a, 11b | piezoelektrische Elemente |
| 12 | scheibenförmiges Element |
| | |
| M | Medium |
| $P_1$-$P_3$ | Prozessgrößen |

| | |
|---|---|
| A | Anregesignal |
| S | Sendesignal |
| $E_A$ | erstes Empfangssignal |
| $E_S$ | zweites Empfangssignal |
| $\Delta\Phi$ | vorgebbare Phasenverschiebung |
| $\rho$ | Dichte des Mediums |
| $v$ | Viskosität des Mediums |
| $v_M$ | Schallgeschwindigkeit im Medium |
| $\tau$ | Laufzeit |
| a | erste Substanz |
| b | zweite Substanz |
| $C_a$ | Konzentration der ersten Substanz |
| $C_b$ | Konzentration der zweiten Substanz |
| L | Abstand zwischen den beiden Gabelzinken |
| H | Dicke des Ansatzes an der Sensoreinheit |

**Patentansprüche**

1. Verfahren zur Bestimmung und/oder Überwachung von zumindest zwei verschiedenen Prozessgrößen ($P_1$, $P_2$) eines Mediums (M), wobei

    - eine Sensoreinheit (2) umfassend eine mechanisch schwingfähige Einheit (4) und zumindest ein piezoelektrisches Element (5) mittels eines Anregesignals (A) zu mechanischen Schwingungen angeregt wird, wobei es sich bei dem Anregesignal (A) um ein elektrisches Signal mit zumindest einer vorgebbaren Frequenz handelt,

    - die mechanischen Schwingungen von der Sensoreinheit (2) empfangen und in ein erstes die mechanischen Schwingungen repräsentierendes, Empfangssignal ($E_A$) umgewandelt werden, **dadurch gekennzeichnet, dass**

    - von derselben Sensoreinheit (2) ein Sendesignal (S) ausgesendet und ein zweites Empfangssignal (Es) empfangen wird, wobei es sich bei dem Sendesignal (S) um ein Ultraschallsignal handelt, und

    - anhand des ersten Empfangssignals ($E_A$) eine erste Prozessgröße ($P_1$) und anhand des zweiten Empfangssignals (Es) eine zweite Prozessgröße ($P_2$) ermittelt wird.

2. Verfahren nach Anspruch 1,

    wobei die Sensoreinheit (2) gleichzeitig mittels des Anregesignals (A) und mittels des Sendesignals (S) beaufschlagt wird, wobei das Anregesignal (A) und das Sendesignal (S) einander überlagert werden, oder

    wobei die Sensoreinheit (2) abwechselnd mittels des Anregesignal (A) und mittels des Sendesignals (S) beaufschlagt wird.

3. Verfahren nach zumindest einem der vorherigen Ansprüche, wobei es sich bei der ersten Prozessgröße ($P_1$) um die Dichte (p) des Mediums (M) und bei der zweiten Prozessgröße ($P_2$) um die Schallgeschwindigkeit ($v_M$) innerhalb des Mediums (M) oder eine daraus abgeleitete Größe handelt.

4. Verfahren nach dem vorherigen Anspruch, wobei anhand der Schallgeschwindigkeit ein Referenzwert für die Dichte ermittelt wird, und wobei der Referenzwert mittels eines aus dem ersten Empfangssignals ermittelten Wertes für die Dichte verglichen wird.

5. Verfahren nach zumindest einem der vorherigen Ansprüche, wobei zumindest eine dritte Prozessgröße ($P_3$), insbesondere die Viskosität ($\eta$) des Mediums (M), bestimmt wird.

6. Verfahren nach zumindest einem der vorherigen Ansprüche, wobei anhand des ersten ($E_A$) und zweiten Empfangssignals (Es) und/oder anhand der ersten ($P_1$) und zweiten Prozessgröße ($P_2$) ermittelt wird, ob sich ein Ansatz an der Sensoreinheit (2) gebildet hat.

7. Verfahren nach zumindest einem der vorherigen Ansprüche, wobei anhand des ersten ($E_A$) und zweiten Empfangssignals (Es) und/oder anhand der ersten ($P_1$) und zweiten Prozessgröße ($P_2$) eine Drift und/oder eine Alterung der Sensoreinheit (2) ermittelt wird.

8. Verfahren nach zumindest einem der vorherigen Ansprüche, wobei das erste ($E_A$) und zweite Empfangssignal (Es), die erste ($P_1$) und zweite Prozessgröße ($P_2$) und/oder ein zeitlicher Verlauf des ersten ($E_A$) und zweiten Empfangssignals (Es) und/oder der ersten ($P_1$) und zweiten Prozessgröße ($P_2$) miteinander verglichen werden.

9. Verfahren nach einem der vorherigen Ansprüche, wobei bei der Bestimmung und/oder Überwachung zumindest einer Prozessgröße ($P_1$-$P_3$) oder bei der Bestimmung einer aus zumindest einer Prozessgröße ($P_1$-$P_3$) und/oder zumindest einem Empfangssignal ($E_A$, Es) abgeleiteten Größe ein Einfluss eines Ansatzes einer Drift und/oder einer Alterung der Sensoreinheit (2) auf das erste ($E_A$) und/oder zweite Empfangssignal (Es) reduziert oder kompensiert wird.

10. Verfahren nach zumindest einem der vorherigen Ansprüche, wobei anhand des ersten ($E_A$) und zweiten Empfangssignals (Es) und/oder anhand der ersten ($P_1$)

und zweiten Prozessgröße ($P_2$) eine erste Konzentration ($C_a$) einer ersten in dem Medium (M) enthaltenen Substanz (a) und eine zweite Konzentration ($C_b$) einer zweiten in dem Medium (M) enthaltenen Substanz (b) ermittelt wird.

11. Verwendung des Verfahrens nach zumindest einem der vorherigen Ansprüche zur Überwachung eines Gärprozesses oder zur Überwachung einer Zuckerinvertierung.

12. Vorrichtung (1) zur Bestimmung und/oder Überwachung einer ersten ($P_1$) und einer zweiten Prozessgröße ($P_2$) eines Mediums (M), umfassend eine Sensoreinheit (2) umfassend eine mechanisch schwingfähige Einheit (4) und zumindest ein piezoelektrisches Element (5), welche Vorrichtung (1) dazu ausgestaltet ist, ein Verfahren nach zumindest einem der vorherigen Ansprüche auszuführen.

13. Vorrichtung (1) nach Anspruch 12, wobei die Sensoreinheit (2) eine mechanisch schwingfähige Einheit (4), und zumindest ein erstes (11a), insbesondere zumindest ein erstes (11a) und ein zweites piezoelektrisches Element (11b), umfasst.

14. Vorrichtung (1) nach Anspruch 13, wobei die mechanisch schwingfähige Einheit (4) eine Schwinggabel mit einem ersten (9a) und einem zweiten Schwingelement (9b) ist, und wobei das erste piezoelektrische Element (10a) zumindest teilweise in einem der beiden Schwingelemente (9a,9b) angeordnet ist, insbesondere wobei das erste piezoelektrische Element (10a) zumindest teilweise in dem ersten Schwingelement (9a) und das zweite piezoelektrische Element (10b) zumindest teilweise in dem zweiten Schwingelement (9b) angeordnet ist.

**Claims**

1. A method for determining and/or monitoring at least two different process variables (P1, P2) of a medium (M), wherein

    - a sensor unit (2) comprising a mechanically vibratable unit (4) and at least one piezoelectric element (5) is excited to mechanically vibrate by means of an excitation signal (A), wherein the excitation signal (A) is an electric signal with at least one predefinable frequency,
    - the mechanical vibrations are received by the sensor unit (2) and converted into a first reception signal ($E_A$) representing the mechanical vibrations, wherein
    - a transmission signal (S) is emitted and a second reception signal (Es) is received by the same sensor unit (2), wherein the transmission signal (S) is an ultrasonic signal, and
    - a first process variable ($P_1$) is ascertained by reference to the first reception signal ($E_A$) and a second process variable ($P_2$) is ascertained by reference to the second reception signal (Es).

2. The method as claimed in claim 1,

    wherein the sensor unit (2) is simultaneously supplied by means of the excitation signal (A) and by means of the transmission signal (S), wherein the excitation signal (A) and the transmission signal (S) are superimposed on one another, or
    wherein the sensor unit (2) is alternately supplied by means of the excitation signal (A) and by means of the transmission signal (S).

3. The method as claimed in at least one of the preceding claims, wherein the first process variable ($P_1$) is the density (p) of the medium (M) and the second process variable ($P_2$) is the speed of sound ($v_M$) within the medium (M) or a variable derived therefrom.

4. The method as claimed in the preceding claim, wherein a reference value for the density is ascertained by reference to the speed of sound, and wherein the reference value is compared by means of a value for the density ascertained from the first reception signal.

5. The method as claimed in at least one of the preceding claims, wherein at least a third process variable ($P_3$), in particular the viscosity ($\eta$) of the medium (M), is determined.

6. The method as claimed in at least one of the preceding claims, wherein it is ascertained whether a deposit has formed on the sensor unit (2) by reference to the first ($E_A$) and the second reception signal ($E_S$) and/or by reference to the first process variable ($P_1$) and the second process variable ($P_2$).

7. The method as claimed in at least one of the preceding claims, wherein a drift and/or an aging of the sensor unit (2) is ascertained by reference to the first reception signal ($E_A$) and the second reception signal ($E_S$) and/or by reference to the first process variable ($P_1$) and the second process variable ($P_2$).

8. The method as claimed in at least one of the preceding claims, wherein the first reception signal ($E_A$) and the second reception signal (Es), the first process variable ($P_1$) and the second process variable ($P_2$) and/or a time profile of the first reception signal ($E_A$) and the second reception signal (Es) and/or of the first process variable ($P_1$) and the second process variable ($P_2$) are compared with one another.

9. The method as claimed in one of the preceding claims,
wherein, when determining and/or monitoring at least one process variable ($P_1$-$P_3$) or when determining a variable derived from at least one process variable ($P_1$-$P_3$) and/or at least one reception signal ($E_A$, Es), an influence of a deposit, a drift and/or an aging of the sensor unit (2) on the first reception signal ($E_A$) and/or the second reception signal (Es) is reduced or compensated for.

10. The method as claimed in at least one of the preceding claims,
wherein a first concentration ($C_a$) of a first substance (a) contained in the medium (M) and a second concentration ($C_b$) of a second substance (b) contained in the medium (M) is ascertained by reference to the first reception signal ($E_A$) and the second reception signal ($E_S$) and/or by reference to the first process variable ($P_1$) and the second process variable ($P_2$).

11. Use of the method as claimed in at least one of the preceding claims for monitoring a fermentation process or for monitoring an inversion of sugar.

12. An apparatus (1) for determining and/or monitoring a first process variable ($P_1$) and a second process variable ($P_2$) of a medium (M), comprising a sensor unit (2) comprising a mechanically vibratable unit (4) and at least one piezoelectric element (5), which apparatus (1) is configured to carry out a method as claimed in at least one of the preceding claims.

13. The apparatus (1) as claimed in claim 12,
wherein the sensor unit (2) comprises a mechanically vibratable unit (4) and at least a first piezoelectric element (11a), in particular at least a first piezoelectric element (11a) and a second piezoelectric element (11b).

14. The apparatus (1) as claimed in claim 13,
wherein the mechanically vibratable unit (4) is a vibrating fork with a first vibrating element (9a) and a second vibrating element (9b), and wherein the first piezoelectric element (10a) is at least partially arranged in one of the two vibrating elements (9a,9b), in particular wherein the first piezoelectric element (10a) is at least partially arranged in the first vibrating element (9a) and the second piezoelectric element (10b) is at least partially arranged in the second vibrating element (9b).

**Revendications**

1. Procédé destiné à la détermination et/ou à la surveillance d'au moins deux grandeurs de process ($P_1$, $P_2$) différentes d'un produit (M), procédé pour lequel

   - une unité de capteur (2) comprend une unité apte à vibrer mécaniquement (4) et au moins un élément piézoélectrique (5), lequel élément est excité en vibrations mécaniques au moyen d'un signal d'excitation (A), le signal d'excitation (A) étant un signal électrique avec au moins une fréquence pouvant être prédéfinie,
   - les vibrations mécaniques sont reçues par l'unité de capteur (2) et sont converties en un premier signal de réception ($E_A$) représentant les vibrations mécaniques, **caractérisé en ce que**
   - un signal d'émission (S) est émis et un deuxième signal de réception (Es) est reçu par la même unité de capteur (2), le signal d'émission (S) étant un signal ultrasonore, et
   - une première grandeur de process ($P_1$) est déterminée à l'aide du premier signal de réception ($E_A$) et une deuxième grandeur de process ($P_2$) est déterminée à l'aide du deuxième signal de réception (Es).

2. Procédé selon la revendication 1,

   pour lequel l'unité de capteur (2) est alimentée simultanément au moyen du signal d'excitation (A) et au moyen du signal d'émission (S), le signal d'excitation (A) et le signal d'émission (S) étant superposés l'un à l'autre, ou
   pour lequel l'unité de capteur (2) est alimentée alternativement au moyen du signal d'excitation (A) et au moyen du signal d'émission (S).

3. Procédé selon au moins l'une des revendications précédentes,
pour lequel la première grandeur de process ($P_1$) est la densité (p) du produit (M) et la deuxième grandeur de process ($P_2$) est la vitesse du son ($v_M$) à l'intérieur du produit (M) ou une grandeur qui en est dérivée.

4. Procédé selon la revendication précédente,

   pour lequel une valeur de référence pour la densité est déterminée à l'aide de la vitesse du son, et
   pour lequel la valeur de référence est comparée au moyen d'une valeur pour la densité déterminée à partir du premier signal de réception.

5. Procédé selon au moins l'une des revendications précédentes,
pour lequel au moins une troisième grandeur de process (P3), notamment la viscosité ($\eta$) du produit (M), est déterminée.

6. Procédé selon au moins l'une des revendications précédentes,
   pour lequel on détermine, à l'aide du premier ($E_A$) et du deuxième signal de réception (Es) et/ou à l'aide de la première ($P_1$) et de la deuxième grandeur de process ($P_2$), si un dépôt s'est formé sur l'unité de capteur (2).

7. Procédé selon au moins l'une des revendications précédentes,
   pour lequel on détermine une dérive et/ou un vieillissement de l'unité de capteur (2) à l'aide du premier ($E_A$) et du deuxième signal de réception (Es) et/ou à l'aide de la première ($P_1$) et de la deuxième grandeur de process ($P_2$).

8. Procédé selon au moins l'une des revendications précédentes,
   pour lequel le premier ($E_A$) et le deuxième signal de réception (Es), la première ($P_1$) et la deuxième grandeur de process ($P_2$) et/ou une évolution dans le temps du premier ($E_A$) et du deuxième signal de réception (Es) et/ou de la première ($P_1$) et de la deuxième grandeur de process ($P_2$) sont comparés entre eux.

9. Procédé selon l'une des revendications précédentes,
   pour lequel, lors de la détermination et/ou de la surveillance d'au moins une grandeur de process ($P_1$-$P_3$) ou lors de la détermination d'une grandeur dérivée d'au moins une grandeur de process ($P_1$-$P_3$) et/ou d'au moins un signal de réception ($E_A$, Es), une influence d'une amorce de dérive et/ou d'un vieillissement de l'unité de capteur (2) sur le premier ($E_A$) et/ou le deuxième signal de réception (Es) est réduite ou compensée.

10. Procédé selon au moins l'une des revendications précédentes,
    pour lequel on détermine, à l'aide du premier ($E_A$) et du deuxième signal de réception (Es) et/ou à l'aide de la première ($P_1$) et de la deuxième grandeur de process ($P_2$), une première concentration ($C_a$) d'une première substance (a) contenue dans le produit (M) et une deuxième concentration ($C_b$) d'une deuxième substance (b) contenue dans le produit (M).

11. Utilisation du procédé selon au moins l'une des revendications précédentes, lequel procédé est destiné à la surveillance d'un process de fermentation ou à la surveillance d'une inversion de sucre.

12. Dispositif (1) destiné à la détermination et/ou à la surveillance d'une première ($P_1$) et d'une deuxième grandeur de process ($P_2$) d'un produit (M), comprenant une unité de capteur (2) comportant une unité apte à vibrer mécaniquement (4) et au moins un élément piézoélectrique (5), lequel dispositif (1) est conçu pour mettre en oeuvre un procédé selon au moins l'une des revendications précédentes.

13. Dispositif (1) selon la revendication 12,
    pour lequel l'unité de capteur (2) comprend une unité apte à vibrer mécaniquement (4), et au moins un premier (11a), notamment au moins un premier (11a) et un deuxième élément piézoélectrique (11b).

14. Dispositif (1) selon la revendication 13,
    pour lequel l'unité apte à vibrer mécaniquement (4) est une fourche vibrante avec un premier (9a) et un deuxième élément vibrant (9b), et
    pour lequel le premier élément piézoélectrique (10a) est disposé au moins partiellement dans l'un des deux éléments vibrants (9a, 9b), le premier élément piézoélectrique (10a) étant notamment disposé au moins partiellement dans le premier élément vibrant (9a) et le deuxième élément piézoélectrique (10b) étant notamment disposé au moins partiellement dans le deuxième élément vibrant (9b).

Fig. 1

Fig. 2a

Fig. 2b

Fig. 2

Fig. 2c

Fig. 2d

Fig. 2

Fig. 3a

Fig. 3b

Fig. 3c

Fig. 3d

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102006034105 A1
- DE 102007013557 A1
- DE 102005015547 A1
- DE 102009026685 A1
- DE 102009028022 A1
- DE 102010030982 A1
- DE 00102010030982 A1
- US 2018224318 A1

- DE 10050299 A1
- DE 102007043811 A1
- DE 10057974 A1
- DE 102006033819 A1
- DE 102015102834 A1
- DE 102016112743 A1
- DE 102012100728 A1
- DE 102017130527 A1